# EUROPEAN PATENT APPLICATION

(11) **EP 4 625 318 A1**
(43) Date of publication of application: **01.10.2025**
(21) Application number: 25165970.2
(22) Date of filing: 25.03.2025
(51) Int. Cl.: G06T 7/00, A61B 6/03

(54) **INFORMATION PROCESSING APPARATUS, INFORMATION PROCESSING METHOD, AND PROGRAM**

(30) Priority: 29.03.2024 JP 2024057939
(71) Applicant: FUJIFILM Corporation, Tokyo 106-8620 (JP)
(72) Inventor: KOKUBUN, Hiroto, Tokyo, 106-8620 (JP)
(74) Representative: Dehns Germany Partnerschaft mbB

(57) **Abstract**

Provided are an information processing apparatus, an information processing method, and a program that can solve a problem of a storage space shortage and a problem of an increase in time required for diagnosis in a case where a plurality of types of images are acquired for the same subject.

An information processing apparatus according to an embodiment of the present disclosure includes a processor configured to: read a first image indicating a three-dimensional form of a subject and a second image indicating a three-dimensional distribution of feature amounts of the subject; extract a diagnosis target from the first image; two-dimensionally develop the diagnosis target based on the first image to create a first development image; project the distribution of the feature amounts included in the diagnosis target onto the first development image based on the second image to create a projection image; and display the projection image on a display device.

## Description

### BACKGROUND OF THE INVENTION

### 1. Field of the Invention

The present disclosure relates to an information processing apparatus, an information processing method, and a program.

### 2. Description of the Related Art

In a radiography apparatus, such as a photon-counting X-ray computed tomography (CT) apparatus, a material discrimination technique is known that discriminates a material included in a subject based on data corresponding to a plurality of energy bands, using the fact that materials have different radiation absorption characteristics. In a case where the material discrimination technique is used, it is possible to acquire a material discrimination image in which a specific material included in the subject has been discriminated, in addition to a normal CT image (for example, see JP2024-032518A).

### SUMMARY OF THE INVENTION

As described above, in a case where a plurality of types of images are acquired for the same subject, a space shortage of a storage for storing the images is likely to occur. In addition, since a diagnosis is performed after a plurality of types of images are displayed side by side on the display device, a long time is required for the diagnosis.

Accordingly, an object of the technology according to the present disclosure is to provide an information processing apparatus, an information processing method, and a program that can solve a problem of a storage space shortage and a problem of an increase in time required for diagnosis in a case where a plurality of types of images are acquired for the same subject.

According to the technology of the present disclosure, there is provided an information processing apparatus comprising a processor configured to: read a first image indicating a three-dimensional form of a subject and a second image indicating a three-dimensional distribution of feature amounts of the subject; extract a diagnosis target from the first image; two-dimensionally develop the diagnosis target based on the first image to create a first development image; project the distribution of the feature amounts included in the diagnosis target onto the first development image based on the second image to create a projection image; and display the projection image on a display device.

Preferably, the processor is configured to: create a second development image in which the diagnosis target has been two-dimensionally developed based on the second image; and combine the first development image and the second development image to create the projection image.

Preferably, the processor is configured to: extract a luminal organ as the diagnosis target; develop the diagnosis target around a tube core line of the luminal organ based on the first image to create the first development image; and develop the diagnosis target around the tube core line based on the second image to create the second development image.

Preferably, the processor is configured to: project a pixel value included in the diagnosis target of the first image onto a first projection plane around the tube core line and then develop the first projection plane to create the first development image; and project a pixel value included in the diagnosis target of the second image onto a second projection plane around the tube core line and then develop the second projection plane to create the second development image.

Preferably, the processor is configured to: project the pixel value included in the diagnosis target of the first image onto the first projection plane using a minimum intensity projection method, a maximum intensity projection method, or an average intensity projection method; and project the pixel value included in the diagnosis target of the second image onto the second projection plane using the minimum intensity projection method, the maximum intensity projection method, or the average intensity projection method.

Preferably, the second image is a material discrimination image showing a material discriminated for the subject.

Preferably, the feature amount is a pixel value of the material discrimination image.

Preferably, the processor is configured to change a color or density of the distribution of the feature amounts projected onto the first development image according to a magnitude of the feature amount.

Preferably, the processor is configured to specify a region in which a magnitude of the feature amount is within a specific range.

Preferably, the material is fat, calcium, a contrast medium, or iron.

Preferably, the processor is configured to calculate a mass, a volume, or a risk parameter of the material in the region and to display a calculated value on the display device.

The feature amount may be a mass, a volume, or a risk parameter of the material on a projection path to the first development image.

The processor may be configured to calculate a total sum of the feature amounts projected onto the first development image or a total risk and to display a calculated value on the display device.

The processor may be configured to display a cross-sectional image corresponding to a position designated on the projection image or a three-dimensional image in a case where the diagnosis target is viewed from an angle corresponding to the position side by side with the projection image on the display device and to use the projection image as a guide for designating the position.

According to the technology of the present disclosure, there is provided an information processing method executed by a processor. The information processing method comprises: reading a first image indicating a three-dimensional form of a subject and a second image indicating a three-dimensional distribution of feature amounts of the subject; extracting a diagnosis target from the first image; two-dimensionally developing the diagnosis target based on the first image to create a first development image; projecting the distribution of the feature amounts included in the diagnosis target onto the first development image based on the second image to create a projection image; and displaying the projection image on a display device.

According to the technology of the present disclosure, there is provided a program causing a processor to execute a process comprising: reading a first image indicating a three-dimensional form of a subject and a second image indicating a three-dimensional distribution of feature amounts of the subject; extracting a diagnosis target from the first image; two-dimensionally developing the diagnosis target based on the first image to create a first development image; projecting the distribution of the feature amounts included in the diagnosis target onto the first development image based on the second image to create a projection image; and displaying the projection image on a display device.

According to the technology of the present disclosure, it is possible to provide an information processing apparatus, an information processing method, and a program that can solve a problem of a storage space shortage and a problem of an increase in time required for diagnosis in a case where a plurality of types of images are acquired for the same subject.

### BRIEF DESCRIPTION OF THE DRAWINGS

Fig. 1 is a diagram showing an outline of a diagnosis support system.
Fig. 2 is a block diagram showing an example of a hardware configuration of an information processing apparatus.
Fig. 3 is a diagram showing a functional configuration of a processor.
Fig. 4 is a diagram showing functions of the processor in more detail.
Fig. 5 is a diagram showing an example of a diagnosis target extracted from a first image.
Fig. 6 is a diagram showing a process of creating a first development image.
Fig. 7 is a diagram schematically showing an example of the first development image.
Fig. 8 is a diagram schematically showing an example of a second development image.
Fig. 9 is a diagram schematically showing an example of a projection image.
Fig. 10 is a diagram showing an example of analysis information.
Fig. 11 is a diagram showing an example of display of the projection image and the analysis information.
Fig. 12 is a diagram showing a flow of a process performed by the processor.
Fig. 13 is a diagram showing a functional configuration of a processor according to a modification example.
Fig. 14 is a diagram showing a cross-sectional image and a three-dimensional image.
Fig. 15 is a diagram showing a flow of a process performed by the processor according to the modification example.

### DESCRIPTION OF THE PREFERRED EMBODIMENTS

Hereinafter, an embodiment of the present disclosure will be described with reference to the drawings.

### First Embodiment

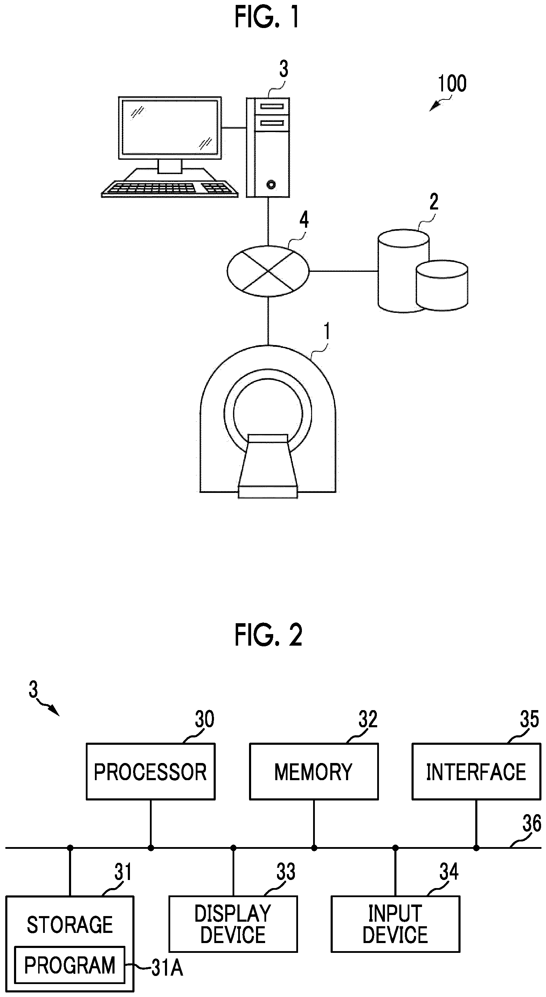

Fig. 1 shows an outline of a diagnosis support system 100 according to an embodiment of the present disclosure. As shown in Fig. 1, the diagnosis support system 100 includes a medical image capturing apparatus 1, an image storage server 2, and an information processing apparatus 3. The medical image capturing apparatus 1, the image storage server 2, and the information processing apparatus 3 are connected in a state in which they can communicate with each other via a network 4. The information processing apparatus 3 is an example of an "information processing apparatus" according to the technology of the present disclosure.

In addition, the information processing apparatus according to the technology of the present disclosure may be a computer included in a console that is connected to the medical image capturing apparatus 1 and controls the medical image capturing apparatus 1 or may be a computer that constitutes a picture archiving and communication system (PACS) together with the image storage server 2. In the present embodiment, the information processing apparatus 3 will be described as the computer included in the PACS.

The medical image capturing apparatus 1 is a photon-counting X-ray CT apparatus or a dual-energy X-ray CT apparatus and can acquire a "CT image" and a "material discrimination image" of the same subject. The CT image is a three-dimensional image (so-called volume image) obtained by stacking a plurality of tomographic images obtained by reconstruction.

The material discrimination image is a three-dimensional image obtained by a material discrimination technique that discriminates a material included in the subject based on data corresponding to a plurality of energy bands, using the fact that materials have different radiation absorption characteristics. The material discrimination image is an image in which a specific material included in the subject has been discriminated. For example, the material discrimination image is an image in which the material has been identified using a K-absorption edge unique to the material. A method for generating the material discrimination image is known in JP2024-032518A and the like. The discriminated material is fat (for example, plaque), calcium, a contrast medium (for example, iodine), or iron. In the present embodiment, the material discrimination image is an image in which "fat" has been discriminated as the material.

Since the CT image is an image based on the intensity of X-rays, the CT image is an image indicating a three-dimensional morphology of the subject. The morphology means the shape of a structure, such as a tissue or an organ, included in the subject. The CT image is an example of a "first image" according to the technology of the present disclosure. Hereinafter, the CT image is referred to as a "first image P1".

The material discrimination image is an image indicating a three-dimensional distribution of feature amounts of the discriminated material. In the present embodiment, the feature amount is a pixel value (that is, a CT value) of the material discrimination image. The unit of the pixel value is HU. For example, the properties of a plaque are determined based on the pixel value, and a material having a pixel value of 30 HU or less is determined to be a "soft plaque". The material discrimination image is an example of a "second image" according to the technology of the present disclosure. Hereinafter, the material discrimination image is referred to as a "second image P2".

The first image P1 and the second image P2 generated by the medical image capturing apparatus 1 are transmitted to the image storage server 2 and stored therein.

The image storage server 2 is a computer that stores and manages various types of data and includes a high-capacity external storage device and database management software. The image storage server 2 communicates with another apparatus via the wired or wireless network 4. Specifically, the image storage server 2 acquires various types of data including the first image P1 and the second image P2 generated by the medical image capturing apparatus 1 via the network 4, stores the acquired data in a storage medium, such as a high-capacity external storage device, and manages the data. In addition, the storage format of image data and the communication between the apparatuses via the network 4 are based on, for example, a protocol such as digital imaging and communication in medicine (DICOM).

Next, a configuration of the information processing apparatus 3 will be described. Fig. 2 shows an example of a hardware configuration of the information processing apparatus 3. The information processing apparatus 3 is a computer, such as a workstation, a server computer, or a personal computer, and comprises a processor 30, such as a central processing unit (CPU), a non-volatile storage 31, and a memory 32 as a temporary storage area. The memory 32 is provided in or externally attached to the processor 30.

In addition, the information processing apparatus 3 comprises a display device 33, such as a liquid crystal display, an input device 34, such as a keyboard and a mouse, and an interface 35, such as a network interface. The processor 30, the storage 31, the memory 32, the display device 33, the input device 34, and the interface 35 are connected to a bus 36.

The storage 31 is implemented by a hard disk drive (HDD), a solid state drive (SSD), or the like. A program 31A is stored in the storage 31 as a storage medium. The processor 30 reads out the program 31A from the storage 31 to the memory 32 and executes a process based on the read-out program 31A.

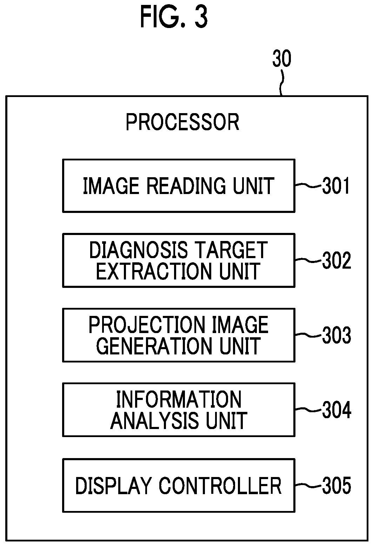

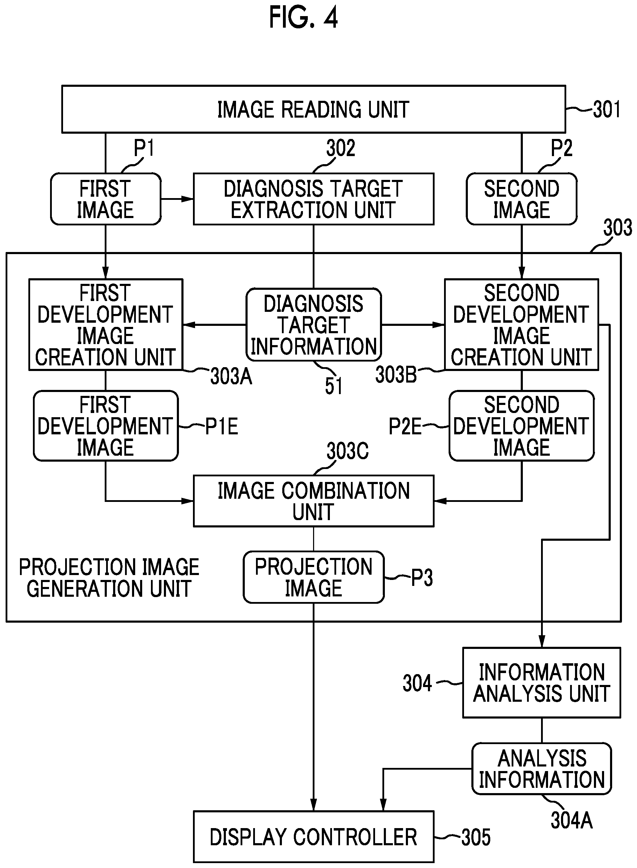

Fig. 3 shows a functional configuration of the processor 30. Fig. 4 shows the functions of the processor 30 in more detail. As shown in Fig. 3, the processor 30 executes the program 31A to function as an image reading unit 301, a diagnosis target extraction unit 302, a projection image generation unit 303, an information analysis unit 304, and a display controller 305. In addition, as shown in Fig. 4, the projection image generation unit 303 includes a first development image creation unit 303A, a second development image creation unit 303B, and an image combination unit 303C.

The image reading unit 301 reads the first image P1 and the second image P2 generated by the medical image capturing apparatus 1 from the image storage server 2.

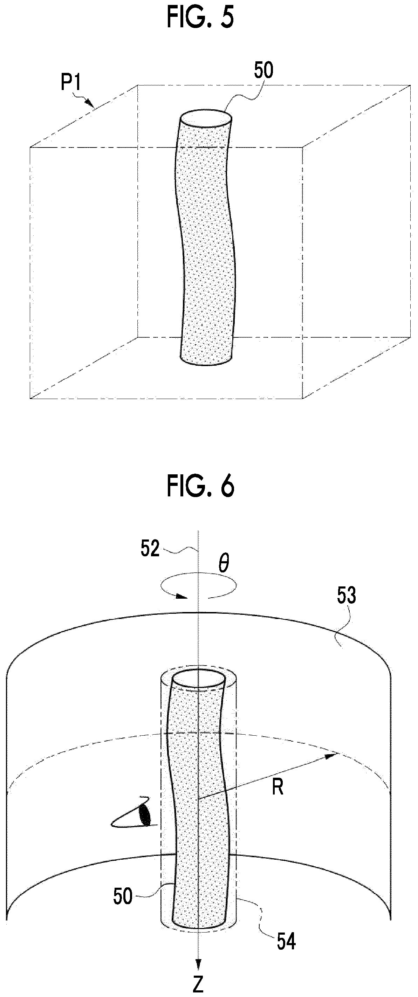

The diagnosis target extraction unit 302 extracts a diagnosis target from the first image P1. For example, the diagnosis target is an organ designated by an operation of the input device 34 by an operator. In the present embodiment, as shown in Fig. 5, a diagnosis target 50 is a luminal organ such as a coronary artery. For example, the diagnosis target extraction unit 302 performs a known three-dimensional image segmentation process on the first image P1 to extract the diagnosis target 50. Information (hereinafter, referred to as diagnosis target information 51), such as the position and shape of the diagnosis target 50 extracted by the diagnosis target extraction unit 302, is supplied to the projection image generation unit 303.

The projection image generation unit 303 two-dimensionally develops the diagnosis target 50 based on the first image P1 to create a first development image P1E and projects the distribution of the feature amounts included in the diagnosis target 50 onto the first development image P1E based on the second image P2 to create a projection image P3.

Specifically, as shown in Fig. 6, the first development image creation unit 303A derives a tube core line 52 of the diagnosis target 50 based on diagnosis target information 51 and develops the diagnosis target 50 around the tube core line 52 based on the first image P1 to create the first development image P1E.

More specifically, the first development image creation unit 303A projects pixel values included in the diagnosis target 50 of the first image P1 onto a projection plane 53 around the tube core line 52 and then develops the projection plane 53 to create the first development image P1E. In this case, the first development image creation unit 303A projects the pixel values included in the diagnosis target 50 onto the projection plane 53 using a minimum intensity projection method, a maximum intensity projection method, or an average intensity projection method. The minimum intensity projection method means a method that projects a minimum value of pixel values on a projection path. The maximum intensity projection method means a method that projects a maximum value of the pixel values on the projection path. The average intensity projection method means a method that projects an average value of the pixel values on the projection path. The projection path is a radial direction (an R direction in Fig. 6) around the tube core line 52. In addition, the projection plane 53 projected by the first development image creation unit 303A corresponds to a "first projection plane" according to the technology of the present disclosure.

In the present embodiment, it is preferable that the first development image creation unit 303A performs the projection using the maximum intensity projection method such that a wall of the luminal organ, which is the diagnosis target 50, is clearly drawn. This is because the wall of the luminal organ has a high X-ray absorbance and has a large pixel value. Further, the first development image creation unit 303A may project, onto the projection plane 53, pixel values in a cylindrical region 54 that has the tube core line 52 as its center and is set with a margin to include the diagnosis target 50.

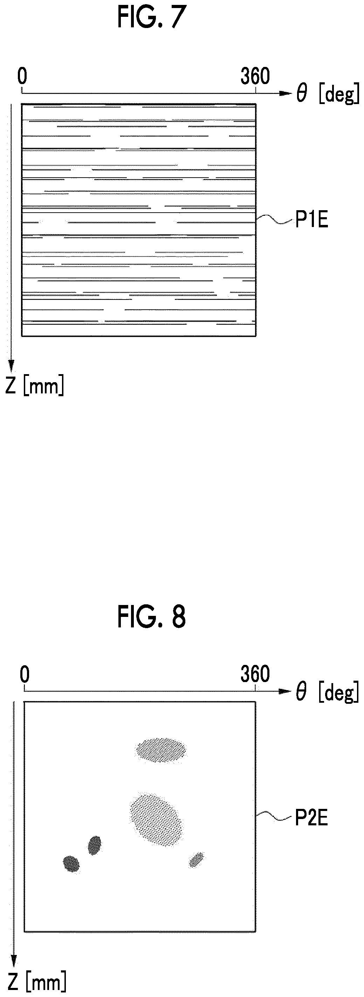

As shown in Fig. 7, the first development image P1E is a two-dimensional image in which a projection value (a minimum value, a maximum value, or an average value) is represented using an angular direction (θ direction in Fig. 6) around the tube core line 52 and a traveling direction (a Z direction in Fig. 6) of the diagnosis target 50 as coordinates.

The second development image creation unit 303B develops the diagnosis target 50 around the tube core line 52 based on the second image P2 to create a second development image P2E. A process performed by the second development image creation unit 303B is the same as the process performed by the first development image creation unit 303A except that the second image P2 is used.

The second development image creation unit 303B projects pixel values included in the diagnosis target 50 of the second image P2 onto the projection plane 53 around the tube core line 52 and then develops the projection plane 53 to create the second development image P2E. In this case, the second development image creation unit 303B projects the pixel values included in the diagnosis target 50 onto the projection plane 53 using the minimum intensity projection method, the maximum intensity projection method, or the average intensity projection method. The projection plane 53 projected by the second development image creation unit 303B corresponds to a "second projection plane" according to the technology of the present disclosure.

In the present embodiment, it is preferable that the second development image creation unit 303B performs the projection using the average intensity projection method such that a soft plaque, which is an abnormal part in the diagnosis target 50, is clearly drawn. The reason is that, since the soft plaque has a low X-ray absorbance, there is a high possibility that a part other than the soft plaque will be drawn in a case where the maximum intensity projection method is used. In addition, the second development image creation unit 303B may project the pixel values in the cylindrical region 54 having the tube core line 52 as its center onto the projection plane 53, similarly to the first development image creation unit 303A.

As shown in Fig. 8, similarly to the first development image P1E, the second development image P2E is a two-dimensional image in which a projection value (a minimum value, a maximum value, or an average value) is represented using the angular direction (θ direction) around the tube core line 52 and the traveling direction (Z direction) of the diagnosis target 50 as the coordinates. The projection value in the second development image P2E indicates the above-described feature amount. In the present embodiment, the properties of the plaque can be discriminated based on the projection value in the second development image P2E. For example, a portion having a projection value of less than 30 HU corresponds to a "soft plaque", a portion having a projection value of 30 HU or more and less than 150 HU corresponds to a "fibrous plaque", and a portion having a projection value of 150 HU or more corresponds to a "calcified plaque".

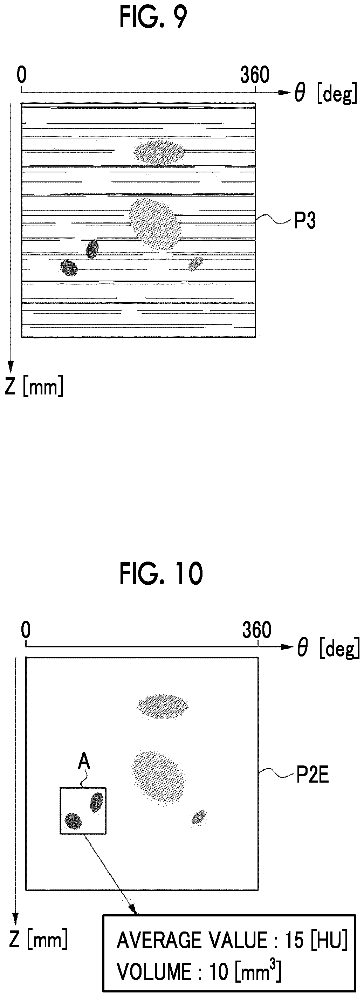

The image combination unit 303C combines the first development image P1E and the second development image P2E to create the projection image P3. Specifically, as shown in Fig. 9, the image combination unit 303C superimposes the first development image P1E and the second development image P2E such that the coordinates are matched with each other to create the projection image P3. The projection image P3 is an image obtained by projecting the distribution of the feature amounts shown in Fig. 8 onto the first development image P1E shown in Fig. 7.

The information analysis unit 304 derives analysis information 304A to be added to and displayed on the projection image P3. In the present embodiment, the information analysis unit 304 specifies a region in which the magnitude of the feature amount is within a specific range. For example, as shown in Fig. 10, the information analysis unit 304 specifies a region A, in which the projection value as the feature amount is within a range of less than 30 HU corresponding to the soft plaque, based on the second image P2. The region A is a region in which the risk of coronary artery rupture is high.

In addition, the information analysis unit 304 calculates the average value of the pixel values, the mass, volume, and risk parameter of the material, and the like in the specified region. The risk parameter is, for example, a calcium score calculated based on the pixel value of the second image P2 and indicates a degree of calcification of the wall of the luminal organ. This calcium score is also referred to as an Agatston score. In the present embodiment, as shown in Fig. 10, the information analysis unit 304 calculates the average value of the pixel values and the volume of the soft plaque in the region A. In the present embodiment, the position and size of the region A, the average value of the pixel values and the volume of the soft plaque in the region A are included in the analysis information 304A.

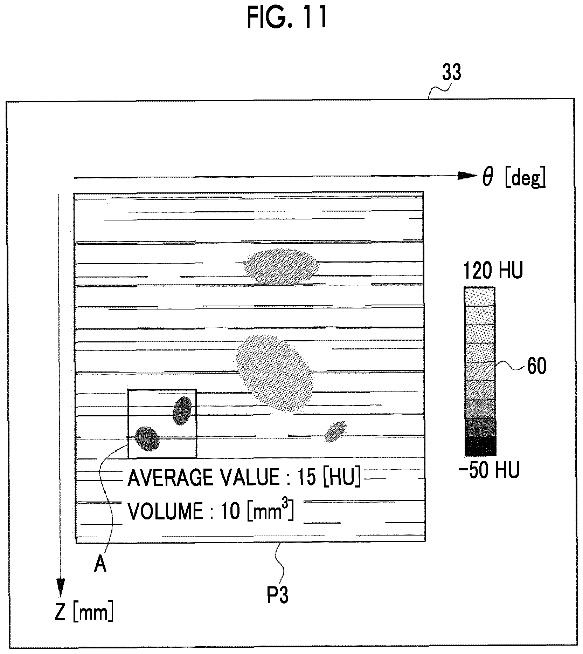

The display controller 305 displays the projection image P3 on the display device 33. In addition, as shown in Fig. 11, the display controller 305 changes the color or density of the distribution of the feature amounts in the projection image P3 according to the magnitude of the feature amount. In the present embodiment, the density is changed according to the magnitude of the projection value as the feature amount. In addition, the display controller 305 displays a chart 60 indicating a relationship between the feature amount and the density on the display device 33 such that the magnitude of the feature amount can be ascertained based on the displayed density of the feature amount.

In addition, the display controller 305 displays the analysis information 304A on the display device 33 in addition to the projection image P3. In the present embodiment, as shown in Fig. 11, the display controller 305 displays a frame indicating the region A, and the average value of the pixel values and the volume of the soft plaque in the region A in the projection image P3 on the display device 33.

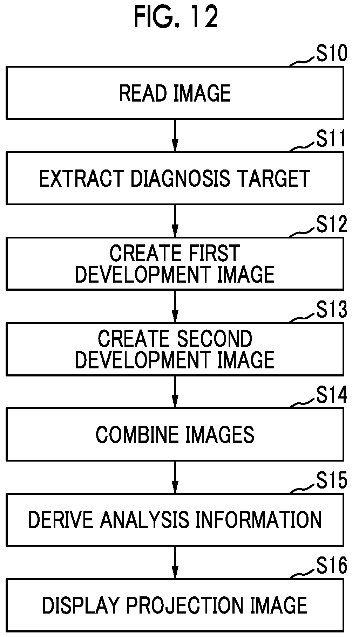

Next, a flow of a process performed by the processor 30 will be described. Fig. 12 shows an example of the flow of the process performed by the processor 30.

First, the image reading unit 301 reads the first image P1 and the second image P2 from the image storage server 2 (Step S10). Then, the diagnosis target extraction unit 302 extracts the diagnosis target 50 from the first image P1 (Step S11). Then, the first development image creation unit 303A creates the first development image P1E based on the first image P1 and the diagnosis target information 51 (Step S12). Then, the second development image creation unit 303B creates the second development image P2E based on the second image P2 and the diagnosis target information 51 (Step S13). In addition, Step S12 and Step S13 may be performed in parallel.

Then, the image combination unit 303C combines the first development image P1E and the second development image P2E to create the projection image P3 (Step S14). Then, the information analysis unit 304 derives the analysis information 304A (Step S15). Further, Step S14 and Step S15 may be performed in parallel.

Then, the display controller 305 displays the projection image P3 and the analysis information 304A on the display device 33 (Step S16).

As described above, in the present embodiment, each of the first image P1 and the second image P2 is not displayed on the display device 33, but the projection image P3 obtained by projecting the distribution of the feature amounts based on the second image P2 onto the first development image P1E created based on the first image P1 is displayed on the display device 33. Therefore, it is not necessary to store the first image P1 and the second image P2 having a large amount of data. As a result, it is possible to solve the problem of a storage space shortage.

In addition, in the present embodiment, since the operator can perform a diagnosis using one projection image P3, it is possible to eliminate the problem of an increase in the time required for diagnosis. Further, in the present embodiment, since the color or density of the distribution of the feature amounts in the projection image P3 is changed according to the magnitude of the feature amount, the operator can visually recognize a region in which an abnormal part, such as a soft plaque, is present. Furthermore, in the present embodiment, since the analysis information 304A is displayed, the operator can easily ascertain a region in which the risk of coronary artery rupture is high, risk information in the region, and the like.

Moreover, in the above-described embodiment, the pixel value of the second image P2 is used as the feature amount. However, the feature amount is not limited thereto. For example, a calculated value of the mass, volume, or risk parameter (for example, a calcium score) of the material on the projection path may be used as the feature amount.

In addition, in the above-described embodiment, the information analysis unit 304 calculates, as the analysis information 304A, the average value of the pixel values, the mass, volume, and risk parameter of the material, and the like in the specified region. However, the information analysis unit 304 may calculate the total sum of the feature amounts or the total risk in the entire projection image P3. In this case, the display controller 305 adds the calculated value of the total sum of the feature amounts or the total risk to the projection image P3 and displays the projection image P3 on the display device 33. For example, the total sum of the feature amounts is an integrated value of the projection values in the entire projection image P3, and the total risk is an integrated value of the calcium scores in the entire projection image P3.

In addition, in the above-described embodiment, the projection image P3 is displayed on the display device 33. However, a cross-sectional image P4 or a three-dimensional image P5 corresponding to a position B designated on the projection image P3 may be displayed on the display device 33. Specifically, the cross-sectional image P4 corresponding to the position B designated on the projection image P3 or the three-dimensional image P5 in a case where the diagnosis target 50 is viewed from an angle corresponding to the position B is displayed side by side with the projection image P3 on the display device 33. In addition, the projection image P3 displayed on the display device 33 can be used as a guide for designating the position B using the input device 34.

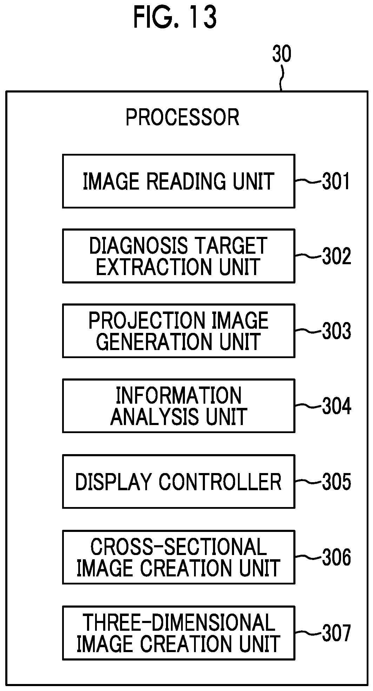

Hereinafter, a modification example of the above-described embodiment in which the projection image P3 can be used as the guide will be described. Fig. 13 shows a functional configuration of a processor 30 according to the modification example. In the present modification example, the processor 30 functions as an image reading unit 301, a diagnosis target extraction unit 302, a projection image generation unit 303, an information analysis unit 304, a display controller 305, a cross-sectional image creation unit 306, and a three-dimensional image creation unit 307. Functions other than the cross-sectional image creation unit 306 and the three-dimensional image creation unit 307 are the same as those in the above-described embodiment.

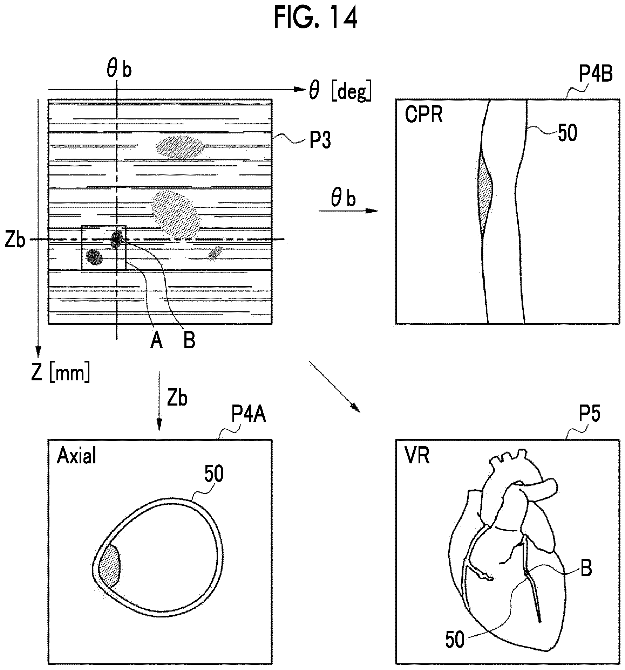

As shown in Fig. 14, in the present modification example, the operator can designate any position B on the projection image P3 displayed on the display device 33 using the input device 34. For example, the operator operates a pointer displayed on the display device 33 with a mouse, sets the pointer to a desired position, and clicks the mouse to designate the position B.

The cross-sectional image creation unit 306 creates the cross-sectional image P4 corresponding to the designated position B based on the first image P1. In the present modification example, the cross-sectional image creation unit 306 creates an axial image P4A and a curved planar reconstruction (CPR) image P4B as the cross-sectional image P4. The axial image P4A is a cross-sectional image obtained by cutting the diagnosis target 50 in a direction orthogonal to the tube core line 52 at a position Zb corresponding to the designated position B in the traveling direction. The CPR image P4B is a cross-sectional image which indicates a cross section along the tube core line 52 and in which an angle θb corresponding to the designated position B is a viewpoint direction.

The three-dimensional image creation unit 307 creates the three-dimensional image P5 in a case where the diagnosis target 50 is viewed from the angle corresponding to the designated position B based on the first image P1. The three-dimensional image P5 is a so-called volume rendering image. The angle corresponding to the designated position B is an angle at which a part corresponding to the designated position B in the three-dimensional image P5 can be visually recognized.

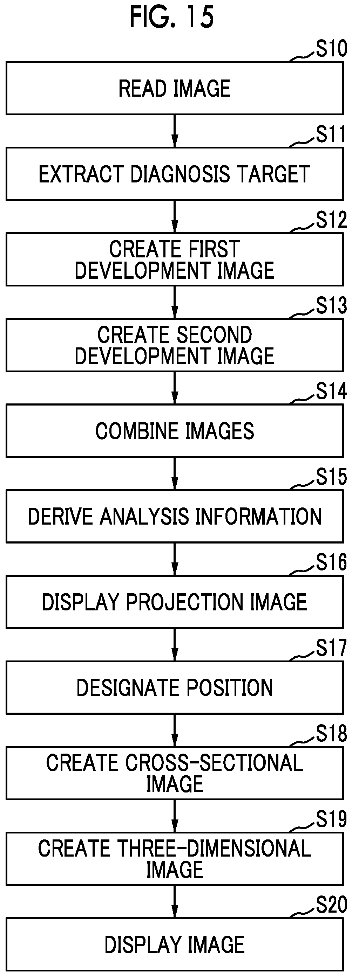

Fig. 15 shows a flow of a process performed by the processor 30 according to the modification example. Steps S10 to S16 are the same as those in the above-described embodiment. In the present modification example, after Step S16, the operator designates any position B on the projection image P3 (Step S17). For example, the operator designates a position where a soft plaque is present. Then, the cross-sectional image creation unit 306 creates the axial image P4A and the CPR image P4B corresponding to the designated position B (Step S18). Then, the three-dimensional image creation unit 307 creates the three-dimensional image P5 in a case where the diagnosis target 50 is viewed from the angle corresponding to the designated position B (Step S19). In addition, Step S18 and Step S19 may be performed in parallel.

Then, the display controller 305 displays the axial image P4A, the CPR image P4B, and the three-dimensional image P5 on the display device 33 (Step S20). For example, the display controller 305 displays the axial image P4A, the CPR image P4B, and the three-dimensional image P5 side by side with the projection image P3 to be adjacent to the projection image P3.

As described above, since the projection image P3 can be used as the guide for designating the position B, the operator can efficiently diagnose a part of interest.

In addition, in the above-described embodiment and modification example, for example, the following various processors can be used as a hardware structure of processing units that execute various processes, such as the image reading unit 301, the diagnosis target extraction unit 302, the projection image generation unit 303, the information analysis unit 304, the display controller 305, the cross-sectional image creation unit 306, and the three-dimensional image creation unit 307. The various processors include, for example, the CPU which is a general-purpose processor executing software (program) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or a combination of two or more processors of the same type or different types (for example, a combination of a plurality of FPGAs and a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-a chip (SoC). As described above, the various processing units are configured by using one or more of the above various processors as the hardware structure.

In addition, specifically, an electric circuit (circuitry) obtained by combining circuit elements, such as semiconductor elements, can be used as the hardware structure of the various processors.

It is possible to ascertain the techniques described in the following supplementary notes from the above description.

### Supplementary Note 1

An information processing apparatus comprising:
a processor configured to:
read a first image indicating a three-dimensional form of a subject and a second image indicating a three-dimensional distribution of feature amounts of the subject;
extract a diagnosis target from the first image;
two-dimensionally develop the diagnosis target based on the first image to create a first development image;
project the distribution of the feature amounts included in the diagnosis target onto the first development image based on the second image to create a projection image; and
display the projection image on a display device.

### Supplementary Note 2

The information processing apparatus according to Supplementary Note 1,
wherein the processor is configured to:
create a second development image in which the diagnosis target has been two-dimensionally developed based on the second image; and
combine the first development image and the second development image to create the projection image.

### Supplementary Note 3

The information processing apparatus according to Supplementary Note 2,
wherein the processor is configured to:
extract a luminal organ as the diagnosis target;
develop the diagnosis target around a tube core line of the luminal organ based on the first image to create the first development image; and
develop the diagnosis target around the tube core line based on the second image to create the second development image.

### Supplementary Note 4

The information processing apparatus according to Supplementary Note 3,
wherein the processor is configured to:
project a pixel value included in the diagnosis target of the first image onto a first projection plane around the tube core line and then develop the first projection plane to create the first development image; and
project a pixel value included in the diagnosis target of the second image onto a second projection plane around the tube core line and then develop the second projection plane to create the second development image.

### Supplementary Note 5

The information processing apparatus according to Supplementary Note 4,
wherein the processor is configured to:
project the pixel value included in the diagnosis target of the first image onto the first projection plane using a minimum intensity projection method, a maximum intensity projection method, or an average intensity projection method; and
project the pixel value included in the diagnosis target of the second image onto the second projection plane using the minimum intensity projection method, the maximum intensity projection method, or the average intensity projection method.

### Supplementary Note 6

The information processing apparatus according to Supplementary Note 4 or 5,
wherein the second image is a material discrimination image showing a material discriminated for the subject.

### Supplementary Note 7

The information processing apparatus according to Supplementary Note 6,
wherein the feature amount is a pixel value of the material discrimination image.

### Supplementary Note 8

The information processing apparatus according to Supplementary Note 6 or 7,
wherein the processor is configured to:
change a color or density of the distribution of the feature amounts projected onto the first development image according to a magnitude of the feature amount.

### Supplementary Note 9

The information processing apparatus according to any one of Supplementary Notes 6 to 8,
wherein the processor is configured to:
specify a region in which a magnitude of the feature amount is within a specific range.

### Supplementary Note 10

The information processing apparatus according to Supplementary Note 9,
wherein the material is fat, calcium, a contrast medium, or iron.

### Supplementary Note 11

The information processing apparatus according to Supplementary Note 9 or 10,
wherein the processor is configured to:
calculate a mass, a volume, or a risk parameter of the material in the region and display a calculated value on the display device.

### Supplementary Note 12

The information processing apparatus according to Supplementary Note 6,
wherein the feature amount is a mass, a volume, or a risk parameter of the material on a projection path to the first development image.

### Supplementary Note 13

The information processing apparatus according to Supplementary Note 6,
wherein the processor is configured to:
calculate a total sum of the feature amounts projected onto the first development image or a total risk and display a calculated value on the display device.

### Supplementary Note 14

The information processing apparatus according to any one of Supplementary Notes 1 to 13,
wherein the processor is configured to:
display a cross-sectional image corresponding to a position designated on the projection image or a three-dimensional image in a case where the diagnosis target is viewed from an angle corresponding to the position side by side with the projection image on the display device; and
use the projection image as a guide for designating the position.

### Explanation of References

1: medical image capturing apparatus
2: image storage server
3: information processing apparatus
4: network
30: processor
31: storage
31A: program
32: memory
33: display device
34: input device
35: interface
36: bus
50: diagnosis target
51: diagnosis target information
52: tube core line
53: projection plane
54: cylindrical region
60: chart
100: diagnosis support system
301: image reading unit
302: diagnosis target extraction unit
303: projection image generation unit
303A: first development image creation unit
303B: second development image creation unit
303C: image combination unit
304: information analysis unit
304A: analysis information
305: display controller
306: cross-sectional image creation unit
307: three-dimensional image creation unit
A: region
B: position
P1: first image
P1E: first development image
P2: second image
P2E: second development image
P3: projection image
P4: cross-sectional image
P4A: axial image
P4B: CPR image
P5: three-dimensional image

## Claims

1. An information processing apparatus comprising:
a processor configured to:
read a first image indicating a three-dimensional form of a subject and a second image indicating a three-dimensional distribution of feature amounts of the subject;
extract a diagnosis target from the first image;
two-dimensionally develop the diagnosis target based on the first image to create a first development image;
project the distribution of the feature amounts included in the diagnosis target onto the first development image based on the second image to create a projection image; and
display the projection image on a display device.

2. The information processing apparatus according to claim 1,
wherein the processor is configured to:
create a second development image in which the diagnosis target has been two-dimensionally developed based on the second image; and
combine the first development image and the second development image to create the projection image.

3. The information processing apparatus according to claim 2,
wherein the processor is configured to:
extract a luminal organ as the diagnosis target;
develop the diagnosis target around a tube core line of the luminal organ based on the first image to create the first development image; and
develop the diagnosis target around the tube core line based on the second image to create the second development image.

4. The information processing apparatus according to claim 3,
wherein the processor is configured to:
project a pixel value included in the diagnosis target of the first image onto a first projection plane around the tube core line and then develop the first projection plane to create the first development image; and
project a pixel value included in the diagnosis target of the second image onto a second projection plane around the tube core line and then develop the second projection plane to create the second development image.

5. The information processing apparatus according to claim 4,
wherein the processor is configured to:
project the pixel value included in the diagnosis target of the first image onto the first projection plane using a minimum intensity projection method, a maximum intensity projection method, or an average intensity projection method; and
project the pixel value included in the diagnosis target of the second image onto the second projection plane using the minimum intensity projection method, the maximum intensity projection method, or the average intensity projection method.

6. The information processing apparatus according to claim 4 or 5,
wherein the second image is a material discrimination image showing a material discriminated for the subject.

7. The information processing apparatus according to claim 6,
wherein the feature amount is a pixel value of the material discrimination image.

8. The information processing apparatus according to any one of the preceding claims,
wherein the processor is configured to:
change a color or density of the distribution of the feature amounts projected onto the first development image according to a magnitude of the feature amount.

9. The information processing apparatus according to any one of the preceding claims,
wherein the processor is configured to:
specify a region in which a magnitude of the feature amount is within a specific range.

10. The information processing apparatus according to any one of claims 6 to 9,
wherein the material is fat, calcium, a contrast medium, or iron.

11. The information processing apparatus according to any one of the preceding claims,
wherein the processor is configured to:
calculate a mass, a volume, or a risk parameter of the material in the region and display a calculated value on the display device.

12. The information processing apparatus according to any one of claims 6 to 11,
wherein the feature amount is a mass, a volume, or a risk parameter of the material on a projection path to the first development image.

13. The information processing apparatus according to any one of the preceding claims,
wherein the processor is configured to:
calculate a total sum of the feature amounts projected onto the first development image or a total risk and display a calculated value on the display device.

14. The information processing apparatus according to any one of the preceding claims,
wherein the processor is configured to:
display a cross-sectional image corresponding to a position designated on the projection image or a three-dimensional image in a case where the diagnosis target is viewed from an angle corresponding to the position side by side with the projection image on the display device; and
use the projection image as a guide for designating the position.

15. An information processing method executed by a processor, the information processing method comprising:
reading a first image indicating a three-dimensional form of a subject and a second image indicating a three-dimensional distribution of feature amounts of the subject;
extracting a diagnosis target from the first image;
two-dimensionally developing the diagnosis target based on the first image to create a first development image;
projecting the distribution of the feature amounts included in the diagnosis target onto the first development image based on the second image to create a projection image; and
displaying the projection image on a display device.

16. A program causing a processor to execute a process comprising:
reading a first image indicating a three-dimensional form of a subject and a second image indicating a three-dimensional distribution of feature amounts of the subject;
extracting a diagnosis target from the first image;
two-dimensionally developing the diagnosis target based on the first image to create a first development image;
projecting the distribution of the feature amounts included in the diagnosis target onto the first development image based on the second image to create a projection image; and
displaying the projection image on a display device.
